# EUROPEAN PATENT SPECIFICATION

(11) **EP 1 259 428 B1**
(45) Date of publication and mention of the grant of the patent: **12.03.2008**
(21) Application number: 01942607.1
(22) Date of filing: 19.01.2001
(51) Int. Cl.: B65B 55/02, A61B 1/00, A61B 17/16

(54) **A METHOD AND APPARATUS FOR INTRODUCING A NON-STERILE COMPONENT INTO A STERILE DEVICE**
VERFAHREN UND VORRICHTUNG ZUM EINFÜHREN EINER NICHT STERILEN KOMPONENTE IN EIN STERILES GERÄT
PROCEDE ET APPAREIL PERMETTANT D'INTRODUIRE UN ELEMENT NON STERILE DANS UN DISPOSITIF STERILE

(30) Priority: 20.01.2000 US 487811
(43) Date of publication of application: 27.11.2002
(73) Proprietor: Bioaccess, Inc., Baltimore, MD 21224 (US)
(72) Inventor: CHAPOLINI, Robert, J., Phoenix, MD 21131 (US); GEISE, Russell, D., Allentown, PA 18104 (US)
(74) Representative: Gudat, Axel
(86) International application number: PCT/US2001/000641
(87) International publication number: WO 2001/053154

(56) References cited:
- WO-A-98/06144
- US-A- 4 919 146
- US-A- 4 991 564
- US-A- 5 078 483
- US-A- 5 105 800
- US-A- 5 569 161
- US-A- 5 812 188
- US-A- 5 971 916

## Description

### (B) CROSS-REFERENCE TO RELATED APPLICATIONS

### (C) STATEMENT REGARDING FEDERALLY SPONSORED RESEARCH

Not Applicable.

### (D) BACKGROUND OF THE INVENTION

### (D1) FIELD OF THE INVENTION

The present invention relates to an apparatus and method for packaging devices having both a sterile component and a non-sterile component, a circumstance that is common to the medical industry, among others. For example, a battery powered surgical drill (the sterile component) includes a removable, rechargeable battery (the non-sterile component).

### (D2) DESCRIPTION OF RELATED ART

Medical and surgical devices continue to become more sophisticated and expensive. Typically, these instruments must be sterilized before use. Instruments that are reusable must be made very durable to withstand repeated sterilizing cycles and this adds considerable manufacturing costs. In order to reduce the cost of these instruments, some manufacturers have provided inexpensive sterile sleeves that completely cover the reusable portion, thus eliminating the need for sterilizing the reusable portion.

The use of motorized devices for therapeutic and medical purposes is very well known in the medical arts. Various types of motorized devices are available for various particular applications. For example, drills and saws are used for the examination and repair in many orthopedic surgical procedures. Coring devices may be used to biopsy or sample bone marrow.

Motorized medical devices provide tremendous advantages in the treatment and repair of many medical conditions, but are typically limited in their capability of repeated use and in the inability or complexity of sterilizing the device between uses. For example, the sterilization of most medical devices presents several substantial disadvantages. First, the device must be manufactured to withstand such sterilization. That is, the device must be formed of materials that will not degrade in the presence of those sterilizing agents in contemporary use. This inherently requires that more expensive materials and manufacturing techniques be utilized and that the sterilization-degradable components be adequately isolated from or compatible with such sterilizing agents.

Second, thorough and proper sterilization of the device requires particular care and is consequently a time-consuming operation. Of course, when the device is being sterilized, it is not available for diagnostic and therapeutic use. Because of the high cost of most motorized medical devices and the consequent lack of availability of spares, the down-time necessitated by sterilization represents lost income for the medical facility. This loss of income increases the cost of performing even simple procedures.

Third, even with the use of such effective sterilizing agents as glutaraldehyde, adequate sterilization of the device cannot be assured. This is of particular concern when the device has a working channel or other such difficult to-clean portions. Furthermore, the use of such toxic materials as glutaraldehyde presents a hazard to the patient in that tissue irritations may result from inadequate flushing of the sterilizing agent from the device. Additionally, special equipment such as a ventilated hood, is required in the use of such toxic sterilization agents, thus increasing the cost of performing such procedures.

For some devices, the currently accepted method of sterilization involves the use of a gas sterilization procedure, typically by exposing the device to ethylene oxide gas for a period of approximately twenty-four hours. As will be recognized, this involves an extended amount of time during which the device is not available for diagnostic and therapeutic use. As with glutaraldehyde, ethylene oxide gas is extremely toxic. Therefore, exposure to personnel must be prevented and traces of the gas must be removed from the device prior to its use to prevent tissue irritation.

U.S. Patent 5,569,161 describes a sterile sleeve for an endoscope. The sleeve interfaces with the distal end of the endoscope and extends over the entire length of the endoscope, thus providing a complete sterile enclosure. It is noted that the sleeve could be packaged as a separate sterile element, thus eliminating the need for on-site sterilization of any component. To use this sterile sleeve, one must grasp the non-sterile endoscope and slide it into the sterile sleeve. This operation typically requires a person with sterile gloves to hold the sterile sleeve and a non-gloved second person to support and guide the non-sterile endoscope into the sleeve.

The Sodem Aseptic Battery Transfer Kit (Sodem Systems, Geneva, Switzerland) discloses a non-sterile battery in a sterile battery housing. The kit requires the use of a sterile centering pin and a sterile external guide, both positioned by sterile personnel, so that non-sterile personnel can introduce a non-sterile battery into the sterile housing. Once the battery is properly positioned in the sterile housing, sterile personnel install a sterile cover, allowing the battery pack to be handled by a person wearing sterile gloves and attached to a battery operated device. This system requires the coordinated efforts of a gloved and non-gloved operator to insert the battery pack into the sterile enclosure.

Cordless surgical drills are frequently used in the operating room. These drills have replaceable battery packs which must be sterilized before use. Steam sterilization can substantially reduce the life expectancy of the battery pack. For example, a battery powered surgical drill typically has a removable, rechargeable battery pack. Although many of these battery packs can be steam sterilized, battery life can be extended by eliminating the sterilizing process and loading the battery into the drill aseptically.

### (E) SUMMARY OF THE INVENTION

The present invention addresses and alleviates the above-mentioned deficiencies in the prior art.

The present invention is an apparatus and method for assembling a device having a sterile component and a non-sterile component. An exemplary apparatus and method includes a non-sterile battery pack positioned into a sterile drill without contaminating the drill.

In one embodiment of the invention, the apparatus comprises a sterile device in sterile packaging, with the sterile device comprising a sleeve or the like having a cover in an open position, said sleeve having an inside portion adapted to communicate with a port in the sterile packaging. The port permits insertion of a non-sterile component, such as a battery, battery pack, and/or motor, into the sleeve of the sterile device. In some embodiments of the invention, the port may be covered with a sterilizable cover; in other embodiments, the entire sterile package or a portion of the package that includes the port may be enclosed in an outer enclosure, such as a bag or the like.

The present invention provides for a means of placing a non-sterile medical device into a sterile sleeve so that the device can be used in a medical procedure that requires aseptic technique. More particularly, the present invention comprises an outer enclosure that contains the sterile sleeve and holds it so that only the inside of the sterile sleeve can be accessed through a separate opening. Insertion of the non-sterile medical device into the invention can be performed by an un-gloved (non-sterile) operator. The outside of the sterile sleeve cannot be inadvertently contaminated during the insertion procedure. After the non-sterile medical device has been inserted, the invention can be opened and the sterile sleeve, with the non-sterile medical device contained within, can be removed by an operator wearing sterile gloves.

The present invention also permits the assembly of a non-sterile component with a sterile component; the two parts, when assembled, form a complete medical device which is sterile on its outer surface. An example of a two component medical device is a disposable surgical drill. Typically, an apparatus such as a disposable drill is packaged as a sterile, single use, disposable medical device. A motor, typically re-usable and non-sterile, must be inserted into the handle of the drill to make it functional. The present invention permits insertion of the non-sterile motor into the sterile drill without any chance of contamination of the outside of the sterile drill. The invention can then be opened and the drill removed by a gloved operator. A hatch door on the sterile drill closes the handle portion and protects from any contact with the non-sterile motor contained inside. At the end of the procedure, the hatch door is opened and the drill placed back in the invention. The invention is closed, thereby containing all surfaces of the drill which have come in contact with the patient. The motor is then removed and retained for future use. The invention with the contaminated drill can then be discarded.

The present invention will foster more innovation in the design of semi-disposable surgical and medical devices and instruments. These designs may combine sophisticated reusable motorized, electronic, and/or mechanical elements adapted to communicate with or engage a sterile device. For example, the hollow interior of a handle or grip of a disposable drill may be shaped (i.e., adapted) to receive a reusable, rechargeable battery.

The present invention will also foster innovative processes in the use and processing of devices enclosed in packaging formed according to the present invention. For example, the process for inserting a non-sterile component into a sterile device in preparation for a surgical procedure will be greatly simplified using packaging according to the present invention. In this way, the coordinated efforts between a gloved personnel and non-gloved personnel can be streamlined and thereby reduce the chance of contamination.

In another embodiment of the invention, the apparatus and method may also include re-packaging a used (e.g., non-sterile and/or contaminated with body fluid) device in the packaging, closing the packaging, and disposing of the package containing the used device. Typically, the disposal process will include disposing of the non-re-useable portions of the device. For example, in preferred embodiments of this aspect of the invention, a battery pack and/or motor may be removed from the device prior to re-packaging. In this way, at the end of the surgical procedure, all patient contact elements are disposed of. This combination of sterile and non-sterile components strikes a balance between the desirability of disposable medical instruments and devices with the reality of medical cost containment.

These, as well as other advantages of the present invention, will be more apparent from the following description and drawings. It is understood that changes in the specific structure shown and described may be made within the scope of the claims without departing from the spirit of the invention.

The detailed description set forth below in connection with the appended drawings is intended as a description of the presently preferred embodiments of the invention, and is not intended to represent the only forms in which the present invention may be constructed or utilized. The description set forth the functions and sequence of steps for constructing and operating the invention in connection with the illustrated embodiments. It is to be understood, however, that the same or equivalent functions and sequences may be accomplished by different embodiments that are also intended to be encompassed within the spirit and scope of the invention.

### (F) DESCRIPTION OF THE DRAWINGS

Fig. 1 is a view of the enclosure with the top partly cut away, showing a sterile, surgical drill in place.
Fig. 2 is a view of the sterile package showing a single access port for insertion of the non-sterile element.
Fig. 3 is a detail view of the enclosure, showing the capture of the open end of the sterile enclosure so as to properly align with the opening in the sterile package.
Fig. 4 is an alternate embodiment of the invention, whereby the alignment of the sterile enclosure with the sterile package is effected by a sleeve extending partway into the open end of the sterile enclosure.
Fig. 5 illustrates the sterile package further enclosed in a sterile pouch which protects the package until used.
Fig. 6 illustrates an alternate embodiment of the invention whereby a peal away cover is used to protect the sterile package until used.
Fig. 7 illustrates a non-sterile motor being loaded into a sterile drill enclosed within the sterile package.
Fig. 8 shows the sterile tray in its opened position, with the combined drill enclosure and motor available to be removed by a gloved operator.
Fig. 9 shows the assembled sterile device (the non-sterile motor completely enclosed within the sleeve), providing a sterile device operable within a sterile field.

### (G) DETAILED DESCRIPTION OF THE INVENTION

The present invention is a packaging apparatus and system for aseptically assembling a sterile device and a non-sterile component of the device. In preferred embodiments of the invention, the assembled device is suitable for use in a sterile environment.

The present invention is also an apparatus for packaging and sterilizing a device comprising an enclosure that maintains a device in a sterile condition and permits insertion of a non-sterile component of the device into a portion of the sterile device while maintaining the device's sterile condition.

The present invention also includes methods for packaging a device having a sterile component and a non-sterile component. An embodiment of the invention includes positioning the device in a package adapted to receive the device and adapted to permit access to a sleeve in the device, sterilizing the device in the package, and inserting a non-sterilized component of the device into the sleeve using an access port in the package. In preferred embodiments of the invention, the method also includes sealing the port prior to sterilizing the package containing the device.

The present invention also includes a method for providing an operable surgical device comprising a sterile device and a non-sterilized component. The method comprises positioning a device in a package adapted to receive the device and having a sealable port, sterilizing the package and device, inserting a non-sterile component of the device into the device through the port, opening the package, closing a cap over the non-sterile component. This process results in an operable device suitable for use in a sterile environment and having both sterile and non-sterile components.

An assembled device according to the invention typically refers to a fully operational device. For example, a typical device would not include a battery and/or a motor, both of which should not be sterilized for reasons noted above. A fully operational device would include all of the elements sufficient for the device to function.

An assembled device according to the invention is suitable for use in a sterile field. A sterile field refers to any location where contamination is undesirable and/or where contamination should or must be maintained at a minimum level. Exemplary environments include but are not limited to medical (e.g., surgery rooms) and electrical (electronics assembly rooms) environments. In a preferred embodiment of the invention, the sterile field is a surgical theater or operating room.

A package or packaging system of the invention may be adapted to receive a device. As used herein adapted to receive refers to forms and sub-structures of the package intended to contact the device and hold the device in position in the package. For example, the package may be molded to conform with the shape of the device. Alternatively, the package may include inserts of the like that have been molded or shaped to conform to the shape of the device. Typical inserts include one or more pieces of foam or the like shaped to conform to the shape of the device or a portion of the device. In some embodiments of the invention, adapted to receive the device includes one or more structures that engage a portion of the device, such as a sleeve. In these embodiments of the invention, these structures engage or form an integral portion of a port in the package.

In accordance with the present invention, the device includes one or more structures adapted to receive a non-sterile component of the functional device. As noted above, the non-sterile component typically refers to one of more batteries and/or a motor. As used herein, adapted to receive a non-sterile component refers to one or more structures in the device suitable for enclosing the non-sterile component and sealing the non-sterile component form the sterile component(s) until the sterility of the functional device is no longer a concern. Exemplary structures suitable for enclosing the non-sterile component include but are not limited to a sleeve, housing, conduit, hollow member, or any other suitable enclosure. In a preferred embodiment of the invention, the sterile device includes a hollow handle, the inside portion of which may be adapted to receive one or more batteries and/or a motor.

A device according to the present invention may also include structures that are configured for and/or are adapted to permit access to the device structure suitable for enclosing the non-sterile component. In a preferred embodiment of the invention, this structure is a port in the package. In a most preferred embodiment of the invention, the port is sealable or coverable. Typical structures for sealing or covering the port include but are not limited to a removable or penetrable seal, an outer package that covers the port, or an outer package that covers an inner package having a port.

As noted above, the invention includes any sterile or sterilizable device that typically require the use or insertion of a non-sterilized component. Exemplary sterile devices include but are not limited to drills, endoscopes, saws, suction devices, accessories to any of the above, and disposables for any of the above. Non-sterilized components include but are not limited to a battery, a battery pack, a motor, a motor/battery assembly, and the like.

As used herein, adapted for communication, communicating, or similar terms refer to any means, structures, or methods for establishing contact between one element of the system and another element. These structures and/or means are well known by practitioners in the art. Exemplary structures are shown in the Figures. For example, a package may include one or more structures that are complementary to the shape of the device and are intended to position the device in a pre-determined place in the package. In a preferred embodiment of the invention, a port in the package may include, be adjacent to, or communicate with a tab or mount or the like that engages a portion of the sleeve on the device. As used herein, connector refers to any structure used to form a joint or to join itself to another piece. Typical connections include but are not limited to mating connections, such as Luer-type, screw-type, friction-type, or connectors that are bonded together.

The present invention, exemplary embodiments of which are shown in the Figures, includes a package 12 suitable for enclosing a device 10 having a portion 11 suitable for engaging and/or enclosing a non-sterilized component of the device. In a preferred embodiment of the invention, package 12 includes a sealable port 13. In a most preferred embodiment of the invention, device 10 also includes a closable cover 14 for capping or closing portion 11.

Each of these elements will now be described in more detail.

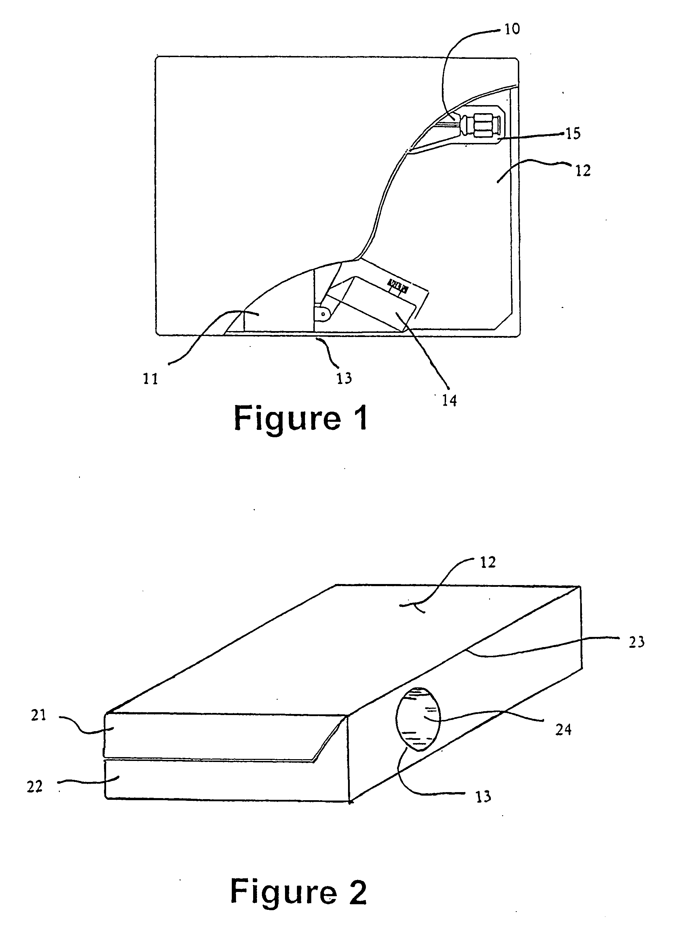

Figures 1 and 2 show packaging or enclosure 12. Enclosure 12 may be constructed of any material compatible with use in a sterile environment, and capable of withstanding sterilization. A wide variety of these containers are already known in the art. For example, these enclosures may be made from plasticized polyvinyl chloride, e.g. PVC plasticized with dioctylphthalate, diethylhexylphthalate, or trioctyltrimellitate; polyolefin, polyurethane, polyester, and/or polycarbonate. It is intended that the present invention is not limited by the type of material used to construct the container.

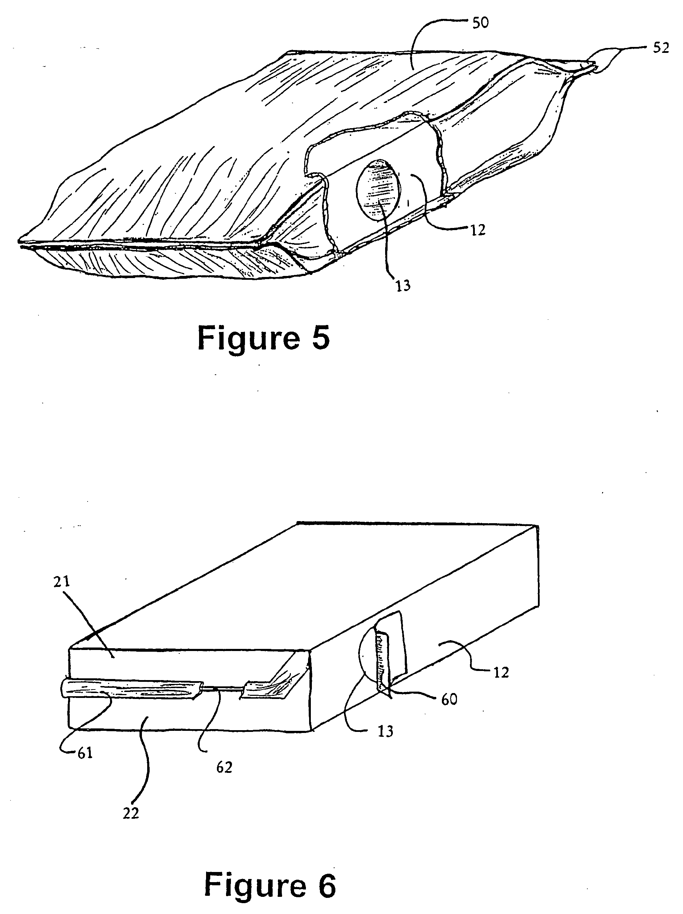

Enclosure 12 may be of any shape and size. One skilled in the art will recognize that the size and shape of device 10 will typically dictate the size and shape of enclosure 12. Enclosure 12 may comprise one or more parts, sections, segments, or the like; preferably enclosure 12 is openable, closable, and/or sealable (see Figure 6). In the embodiment of the invention shown in Figure 2, enclosure 12 may comprise an upper section 21 and a lower section 22 molded or joined by a hinge 23 region. As illustrated, section 21 and section 22 matingly engage when in a closed position.

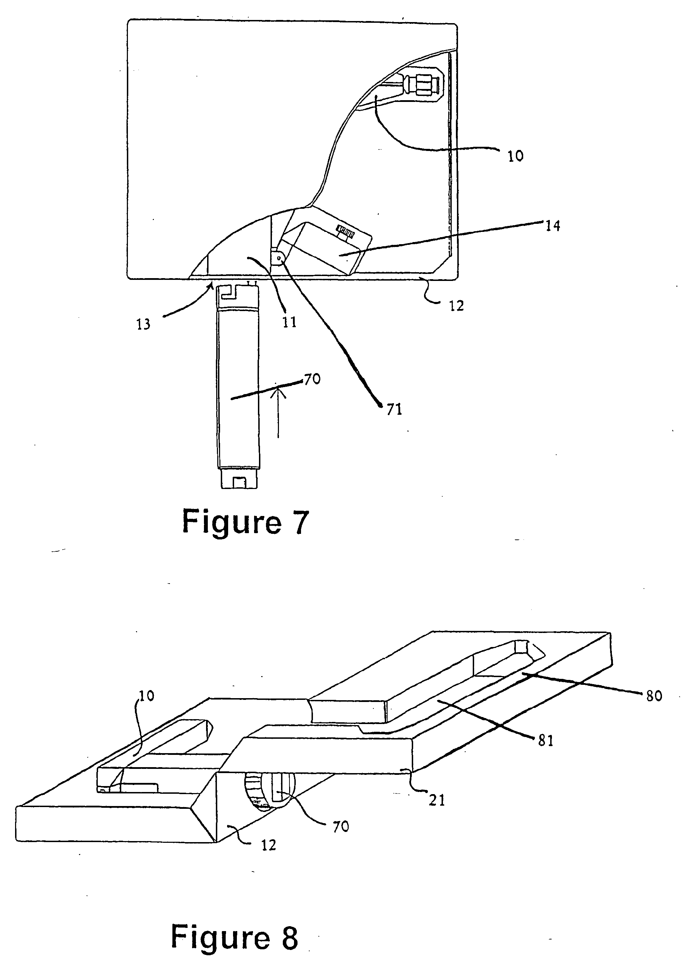

Enclosure 12 may be adapted to receive device 10. Preferably enclosure 12 is adapted by forming portions of the enclosure to engage and/or secure device 10 in place. One skilled in the art will recognize that a variety of structures, materials, and systems may be employed to position device 10 in enclosure 12. In a preferred embodiment of the invention, an interior section of enclosure 12 is molded to the shape of device 10. For example, Figure 1 shows a void or cavity 15 adapted to receive device 10. Alternatively, as shown in Figure 8, enclosure 12 may include one or more inserts 80 configured to be positioned within enclosure 12 and configured to receive or engage device 10. As illustrated in Figure 8, insert 80 may be shaped or configured with one or more cavities to matingly engage or secure device 10. One or more inserts 80 may be formed of any material that is sterilizable, and is preferably formed of a foam or spongy material.

As shown in Figure 2, enclosure 12 comprises a port 13 positioned adjacent to, contiguous with, or proximate an inside portion and relative aperture 24 of sleeve 11 of device 10. In the most preferred embodiments of the invention, port 13 is sealable. Any structure or means for sealing port 13 may be used in accordance with the present invention. Exemplary structures include but are not limited to an outer or second enclosure 50 that encloses package or first enclosure 12 including port 13 (see Figure 5), a covering 60 for sealing port 13 (see Figure 6), or both.

Outer enclosure or pouch 50 prevents contamination of the inside of the device contained within enclosure 12 until the device is needed. Pouch 50 may be constructed of any material typically used to protect sterile surgical and medical instruments from contamination. The pouch 50 is preferably sealable and/or completely sealed along its edges. In Figure 5 pouch 50 is shown partly cut away to reveal enclosure 12 and port 13 contained within.

Figure 6 shows an alternate structure and method for providing a contamination barrier for the inside of device 10. In the illustrated embodiment of the invention, upper section 21 and lower section 22 of enclosure 12 may be sealed with a gasket or removable tape 61 along the seam 62 between upper section 21 and lower section 22. Further, port 13 of enclosure 12 may be sealed along the edge of port 13 using cover 60 or the like. Cover 60 may be a removable strip or the like or may be penetratable membrane or the like. The removable cover 60 is shown partially pealed away. As described in more detail below, when removable seal 60 is removed or penetrated, access to the inside of sleeve 11 of device 10 is permitted. In a preferred embodiment of the invention, the combination of the removable tape 61 and removable cover 60 prevents contamination of the device 10 positioned in enclosure 12 until it is ready for use.

As noted above, enclosure 12 may comprise an integral structure, two or more mated and/or matable sections, or combinations thereof. A preferred embodiment of the invention comprises enclosure 12 comprising upper 21 and lower 22 sections (see Figure 2). Figure 2 shows enclosure 12 in its closed position. A releasable capture mechanism , not shown, keeps the upper section 21 and the lower section 22 matingly engaged until the enclosed device 10 is needed. In the illustrated embodiment, enclosure 12 is openable, preferably using a hinge 23 or the like to provide access to the device 10 contained within.

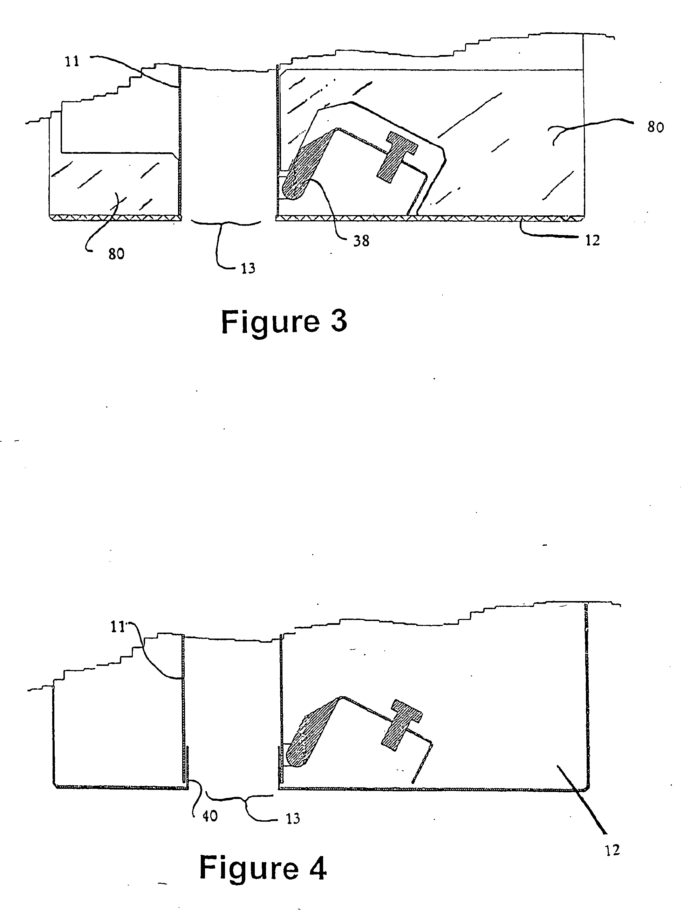

In the embodiments of the invention shown in Figures 1, 3, and 4, sleeve 11 of device 10 is positioned in alignment with, adjacent to, and/or engaged with port 13. In a most preferred embodiment of the invention, port 13 permits access to an inner portion of sleeve 11 while enclosure 12 is closed. In the illustrated embodiments, sleeve 11 is a hollow handle of device 10. As shown, sleeve 11 of device 10 is positioned in the sterile enclosure 12 so that an open end of the handle is aligned with port 13 in the side of the enclosure 12. One skilled in the art will readily recognize that a variety of structures and means may be used to properly position the open end of sleeve 11 in relation to port 13. Figure 1 illustrates an exemplary configuration in which a cavity 15, not adjacent port 13, positions sleeve 11 adjacent port 13. Figure 3 shows an exemplary configuration is which one or more inserts 80 that position sleeve 11 adjacent port 13.

Figure 4 shows an exemplary configuration is which port 13 and/or enclosure 12 include an alignment sleeve 40, seat, guide, tab, or the like that aligns or matingly engages an inner portion of sleeve 11 to position sleeve 11 in relation to port 13. The present invention includes using one or more of the these configurations to position the inside of sleeve 11 in relation to port 13.

The present invention also includes a method for combining a non-sterile component of a device with a sterile component of the device while maintaining the sterility of the sterile component. Exemplary structures that illustrate the methods of the invention are shown in Figures 7-9.

As is well known to practitioners in the art, the protocols for maintaining the sterility of a device 10 until it is operable and/or ready for use are varied. Typically, these protocols involve a single technician performing certain actions while not sterile (in medical parlance, un-gloved) and other actions while sterile (e.g., gloved), or the coordinated actions between a sterile technician and a non-sterile technician. The essence of these actions is that a non-sterile technician should not touch a sterile device or enter a sterile field, and likewise, a sterile technician should not touch a non-sterile device. The various embodiments of the present invention accommodate all of these protocols.

In accordance with the apparatus and method of the present invention, the portion of the device 10 that needs to be sterilized is placed in enclosure 12. At this point in the process, port 13 of enclosure 12 may be open or sealed, but is preferably sealed with covering 60 or tape 61. Enclosure 12 is then closed and preferably sealed, as described above. The closed enclosure 12 may then be sterilized, but it is preferable to place the closed enclosure 12 in an outer pouch 50 prior to sterilization. If port 13 does not have a seal or cover, enclosure 12 must be placed in an outer pouch 50 prior to sterilization. Enclosure 12 containing a portion of device 10 may then be sterilized.

Once sterilization is complete pouch 50 containing enclosure 12 and device 10 may be stored until ready for use. When ready for use, a non-sterile technician opens pouch 50 in a controlled environment, such as a procedure room, a clean room, or an operating room. The non-sterile technician may then remove covering 60 and insert a the non-sterile component 70 of device 10 into the sleeve 11 of device 10. The non-sterile component 70 is one or more elements needed to make device 10 functional or operable, but which may be deleteriously affected by exposure to sterilizing agents. Non-sterile components include but are not limited to one or more batteries, one or more motors, a battery pack, or combinations thereof.

In some embodiments of the invention, the non-sterile technician will then remove cover 60 from port 13 and slide non-sterile component 60 into sleeve 11. In other embodiments of the invention, the non-sterile technician will push the non-sterile component through a penetratable seal covering port 13. These actions are illustrated in Figure 7. In this embodiment of the invention, the top of sterile enclosure 12 is shown partially cut away, revealing the enclosed device 10, such as a sterile surgical drill. It should be noted that the sterile enclosure 12 can be handled by a non-gloved operator without contaminating the outside of enclosed sterile device 10 because the portion of enclosure 12 covering device 10 is still closed or sealed. The same non-gloved operator can also insert the non-sterile component 70 into the sterile device 10. In a preferred embodiment, the non-sterile component 70 may be locked into position in the sterile device 10.

The non-sterile technician then opens enclosure 10, as is shown in Figure 8. The upper section 21 of enclosure 12 has been folded back to provide access to sterile device 10. At this point, a sterile or gloved technician may touch device 10, now containing a non-sterile component. The sterile technician should limit contact to only the inside of the sterile package 12 and the sterile device 10.

In some embodiments of the invention, device 10 is now functional or operable by a sterile or gloved person. In alternative and preferred embodiments of the invention, device 10 or sleeve 11 includes a cover 14, sterilized at the same time that device 10 is sterilized, that can then be closed over any protruding or uncovered portion of non-sterile component 70. One skilled in the art will recognize that cover 14 can seal sleeve 11 using a variety of structures and mechanisms. Exemplary structures include but are not limited to a friction fit or snap fit, or a spring-loaded assembly between cover 14 and sleeve 11, and/or a hinged arrangement between cover 14 and sleeve 11. The preferred embodiment for this aspect of the invention is illustrated in Figures 1, 7 and 9, and includes a hinge 71. In a most preferred embodiment of the invention, cover 14 includes a thumb screw 90 or the like for ascertaining that the cover 14 remains closed or sealed over non-sterile component 70.

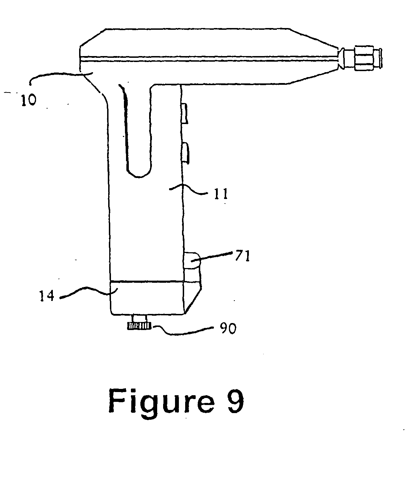

The sterile or gloved technician may then close cover 14 and tighten screw 90. Device 10 containing non-sterile component 70 is now fully operational and suitable for handling and operating by a sterile or gloved person (see Figure 9).

After use, the non-sterile component can be removed from the device 10 and reused one or more times.

In a further embodiment of the invention, once the operator has finished using device 10, it may be re-packaged in enclosure 12 to insure that other are protected from the now-contaminated device. In this embodiment of the invention, the intent is to retain the contamination inside the enclosure, close the enclosure, and transport the closed enclosure containing the_contaminated device to a treatment center. Typically, any re-useable components of the device, or components that should not be sterilized, are separated from the device. The remainder of the device, e.g., the sleeve, handle, and/or housing, may be disposed or may be re-sterilized prior to use again.

Although the present invention has been described in terms of a particular preferred embodiments, it is not limited to those embodiments. Alternative embodiments, examples, and modifications which would still be encompassed by the invention may be made by those skilled in the art, particularly in light of the foregoing teachings.

## Claims

1. An apparatus (12, 10) for aseptically assembling a sterile component (10) and a non-sterile component (70) said apparatus comprising a sterile (11) sleeve having a non-sterile aperture (24), and packaging material (12) enclosing said sterile sleeve (11) and having a non-sterile port (13) communicating with said non-sterile aperture (24).

2. The apparatus of claim 1 wherein said sterile sleeve (11) includes a non-sterile inside portion (24) said inside portion communicating with said non-sterile aperture (24).

3. The apparatus of claim 1 wherein said sterile sleeve (11) further comprises a cover (14) for closing said non-sterile aperture (24).

4. The apparatus of claim 1 wherein the sterile component (10) and the non-sterile component (70) comprise a medical device.

5. The apparatus of claim 4 wherein the medical device is a drill.

6. The apparatus of claim 5 wherein the drill comprises a sterile sleeve (11) and a non-sterile motor (70) adapted to communicate with an inside portion (24) of said sterile sleeve (11).

7. The apparatus of claim 5 wherein the drill comprises a sterile sleeve (11) and a non-sterile battery assembly (70) adapted to communicate with an inside portion (24) of said sterile sleeve.

8. The apparatus of claim 4 wherein the medical device is an endoscope.

9. The apparatus of claim 1 wherein the sterile component is disposable.

10. The apparatus of claim 1 wherein the non-sterile component is non-disposable.

11. The apparatus of claim 1 wherein the packaging material is openable.

12. A method for packaging a device (10) having a sterile component (11) and a non-sterile component (70) comprising providing a device (10) having a sterile sleeve (11) and a non-sterile aperture (24) communicating with an inside portion (24) of said sleeve; providing packaging material (12) enclosing an outside portion of said sleeve (11), said packaging material having a port (13) communicating with the inside portion (24) of said sleeve; placing a non-sterile component (70) of said device into the inside portion (24) of said sleeve; closing said aperture (24); and opening said packaging (12).

13. The method of claim 12 wherein placing a non-sterile component (70) of said device into the inside portion (24) of said sleeve comprises maintaining an outside portion of said sleeve (11) in a sterile condition.

14. The method of claim 12 wherein placing a non-sterile component (70) said device into the inside portion of said sleeve (11) is conducted under non-sterile conditions.

15. A method of assembling a device (10) having a non-sterile component (70) and a sterile component (11) comprising providing a device (10) having a sleeve, said sleeve (11) comprising a sterile outer portion (11) and a non-sterile inside portion (24); providing an enclosure (12) adapted to communicate with said device and adapted to maintain the sterility of the outside portion (11) of said sleeve, said enclosure having a port (13) for communicating with an inside portion (24) of said sleeve; and positioning said non-sterile component (70) in said sleeve (11) by passing said non-sterile component (70) through said port (13).

16. An aseptic packaging system comprising an openable package (12) adapted to position an aseptic device (10) in the package (12), said aseptic device (10) comprising a sterile outer portion (11) and a non-sterile inner portion (24); said package comprising a port (13) adapted to communicate with the non-sterile inner portion (24) of said device.

## Patentansprüche

1. Vorrichtung (12, 10) zum aseptischen Zusammenbau einer sterilen Komponente (10) und einer nicht-sterilen Komponente (70) der Vorrichtung, umfassend eine sterile Hülse (11) mit einer nicht-sterilen Öffnung (24) und Verpackungsmaterial (12), welches die sterile Hülse (11) umschließt und einen nicht-sterilen Durchlass (13) aufweist, welcher mit der nicht-sterilen Öffnung (24) in Verbindung steht.

2. Vorrichtung nach Anspruch 1, wobei die sterile Hülse (11) einen nicht-sterilen innen liegenden Bereich (24) aufweist, wobei der innen liegende Bereich mit der nicht-sterilen Öffnung (24) in Verbindung steht.

3. Vorrichtung nach Anspruch 1, wobei die sterile Hülse (11) des Weiteren eine Abdeckung (14) zum Verschließen der nicht-sterilen Öffnung (24) aufweist.

4. Vorrichtung nach Anspruch 1, wobei die sterile Komponente (10) und die nicht-sterile Komponente (70) ein medizinisches Instrument aufweisen.

5. Vorrichtung nach Anspruch 4, wobei das medizinische Instrument ein Bohrer ist.

6. Vorrichtung nach Anspruch 5, wobei der Bohrer eine sterile Hülse (11) und einen nicht-sterilen Motor (70), welcher dazu angepasst ist, mit einem innen liegenden Bereich (24) der sterilen Hülse (11) in Verbindung zu stehen, aufweist.

7. Vorrichtung nach Anspruch 5, wobei der Bohrer eine sterile Hülse (11) und eine nicht-sterile Batteriebaugruppe (70), welche angepasst ist, um mit einem innen liegenden Bereich (24) der sterilen Hülse in Verbindung zu stehen, aufweist.

8. Vorrichtung nach Anspruch 4, wobei das medizinische Instrument ein Endoskop ist.

9. Vorrichtung nach Anspruch 1, wobei die sterile Komponente absetzbar ist.

10. Vorrichtung nach Anspruch 1, wobei die nicht-sterile Komponente nicht absetzbar ist.

11. Vorrichtung nach Anspruch 1, wobei das Verpackungsmaterial geöffnet werden kann.

12. Verfahren zur Verpackung eines Instrumentes (10) mit einer sterilen Komponente (11) und einer nicht-sterilen Komponente (70), welches umfasst die Bereitstellung eines Instrumentes (10) mit einer sterilen Hülse (11) und einer nicht-sterilen Öffnung (24), welche mit einem innen liegenden Bereich (24) der Hülse in Verbindung steht; Bereitstellung von Verpackungsmaterial (12), welches einen außen liegenden Bereich der Hülse (11) umschließt, wobei das Verpackungsmaterial einen Durchlass (13) aufweist, welcher mit dem innen liegenden Bereich (24) der Hülse in Verbindung steht; Anordnung einer nicht-sterilen Komponente (70) des Instrumentes in den innen liegenden Bereich (24) der Hülse; Schließen der Vorrichtung (24); und Öffnen der Verpackung (12).

13. Verfahren nach Anspruch 12, wobei die Anordnung der nicht-sterilen Komponente (70) des Instrumentes in dem innen liegenden Bereich (24) der Hülse eine Beibehaltung eines außen liegenden Bereichs der Hülse (11) in einem sterilen Zustand umfasst.

14. Verfahren nach Anspruch 12, wobei die Anordnung einer nicht-sterilen Komponente (70) des Instrumentes in den innen liegenden Bereich der Hülse (11) unter nicht-sterilen Bedingungen durchgeführt wird.

15. Verfahren zum Zusammenbau eines Instrumentes (10) mit einer nicht-sterilen Komponente (70) und einer sterilen Komponente (11) umfassend die Bereitstellung eines Instrumentes (10) mit einer Hülse, wobei die Hülse (11) einen sterilen äußeren Bereich (11) und einen nicht-sterilen inneren Bereich (24) aufweist; Bereitstellung einer Umhüllung (12), welche dazu angepasst ist, mit dem Instrument in Verbindung zu stehen, und angepasst ist, die Sterilität des außen liegenden Bereichs (11) der Hülse aufrecht zu erhalten, wobei die Umhüllung einen Durchlass (13) zur Verbindung mit einem innen liegenden Bereich (24) der Hülse aufweist; und Positionierung der nicht-sterilen Komponente (70) in der Hülse (11) durch Durchführen der nicht-sterilen Komponente (70) durch den Durchlass (13).

16. Aseptisches Verpackungssystem umfassend eine öffenbare Verpackung (12), welche angepasst ist, ein aseptisches Instrument (10) in der Verpackung (12) anzuordnen, wobei das aseptische Instrument (10) einen sterilen äußeren Bereich (11) und einen nicht-sterilen inneren Bereich (24) aufweist; und die Verpackung einen Durchlass (13) aufweist, der angepasst ist, um mit dem nicht-sterilen inneren Bereich (24) des Instrumentes in Verbindung zu stehen.

## Revendications

1. Appareil (12, 10) pour assembler de manière aseptique un composant stérile (10) et un composant non stérile (70) comprenant un manchon stérile (11) ayant une ouverture non stérile (24) et un matériau d'emballage (12) renfermant ledit manchon stérile (11) et ayant un orifice non stérile (13) communiquant avec ladite ouverture non stérile (24).

2. Appareil selon la revendication 1, dans lequel ledit manchon stérile (11) inclut une partie intérieure non stérile (24), ladite partie intérieure communiquant avec ladite ouverture non stérile (24).

3. Appareil selon la revendication 1, dans lequel ledit manchon stérile (11) comprend en outre un opercule (14) pour fermer ladite ouverture non stérile (24).

4. Appareil selon la revendication 1, dans lequel le composant stérile (10) et le composant non stérile (70) comprennent un dispositif médical.

5. Appareil selon la revendication 4, dans lequel le dispositif médical est un perforateur.

6. Appareil selon la revendication 5, dans lequel le perforateur comprend un manchon stérile (11) et un moteur non stérile (70) adapté pour communiquer avec une partie intérieure (24) dudit manchon stérile (11).

7. Appareil selon la revendication 5, dans lequel le perforateur comprend un manchon stérile (11) et un ensemble de pile(s) non stérile (70) adapté pour communiquer avec une partie intérieure (24) dudit manchon stérile.

8. Appareil selon la revendication 4, dans lequel le dispositif médical est un endoscope.

9. Appareil selon la revendication 1, dans lequel le composant stérile est jetable.

10. Appareil selon la revendication 1, dans lequel le composant non stérile n'est pas jetable.

11. Appareil selon la revendication 1, dans lequel le matériau d'emballage peut s'ouvrir.

12. Procédé pour emballer un dispositif (10) ayant un composant stérile (11) et un composant non stérile (70) comprenant la fourniture d'un dispositif (10) ayant un manchon stérile (11) et une ouverture non stérile (24) communiquant avec une partie intérieure (24) dudit manchon ; la fourniture d'un matériau d'emballage (12) renfermant une partie extérieure dudit manchon (11), ledit matériau d'emballage ayant un orifice (13) communiquant avec la partie intérieure (24) dudit manchon ;le placement d'un composant non stérile (70) dudit dispositif dans la partie intérieure (24) dudit manchon ; la fermeture de ladite ouverture (24) et l'ouverture dudit emballage (12).

13. Procédé selon 1a revendication 12, dans lequel le placement d'un composant non stérile (70) dudit dispositif dans la partie intérieure (24) dudit manchon comprend le maintien d'une partie extérieure dudit manchon (11) dans un état stérile.

14. Procédé selon la revendication 12, dans lequel le placement d'un composant non stérile (70) dudit dispositif dans la partie intérieure dudit manchon (11) est effectué dans des conditions non stériles.

15. Procédé d'assemblage d'un dispositif (10) ayant un composant non stérile (70) et un composant stérile (11) comprenant la fourniture d'un dispositif (10) ayant un manchon, ledit manchon (11) comprenant une partie extérieure stérile (11) et une partie intérieure non stérile (24) ; la fourniture d'une enveloppe (12) adaptée pour communiquer avec ledit dispositif et adaptée pour conserver la stérilité de la partie extérieure (11) dudit manchon, ladite enveloppe ayant un orifice (13) pour communiquer avec une partie intérieure (24) dudit manchon et le positionnement dudit composant non stérile (70) dans ledit manchon (11) en faisant passer ledit composant non stérile (70) à travers ledit orifice (13).

16. Système d'emballage aseptique comprenant un emballage (12) pouvant s'ouvrir, adapté pour placer un dispositif aseptique (10) dans l'emballage (12), ledit dispositif aseptique (10) comprenant une partie extérieure stérile (11) et une partie intérieure non stérile (24) ; ledit emballage comprenant un orifice (13) adapté pour communiquer avec la partie intérieure non stérile (24) dudit dispositif.
